Europäisches Patentamt

European Patent Office

Office européen des brevets

(11) Veröffentlichungsnummer: **0 341 693 B1**

(12) **EUROPÄISCHE PATENTSCHRIFT**

(45) Veröffentlichungstag der Patentschrift: **02.12.92**

(21) Anmeldenummer: **89108426.1**

(22) Anmeldetag: **10.05.89**

(51) Int. Cl.⁵: **C07C 239/20**

(54) Verfahren zur Herstellung von O-substituierten Hydroxylaminen.

(30) Priorität: **11.05.88 CH 1778/88**

(43) Veröffentlichungstag der Anmeldung:
**15.11.89 Patentblatt 89/46**

(45) Bekanntmachung des Hinweises auf die
Patenterteilung:
**02.12.92 Patentblatt 92/49**

(84) Benannte Vertragsstaaten:
**AT BE CH DE FR GB IT LI NL SE**

(56) Entgegenhaltungen:
**DD-A- 221 456**

**HOUBEN WEYL "Methoden der organischen Chemie", 4. Auflage, Band X/1 "Stick- -stoffverbindungen I", Teil 1, 1971 GEORG THIEME VERLAG, Stuttgart Seiten 1181-1185**

**CHEMICAL ABSTRACTS, Band 64, Nr. 12, 6. Juni 1966, Columbus, Ohio, USA G.BARGIGIA "Synthesis of 0-substituted hadroxylamines (RONH2) from alcs. and isohydroxylamine monosulfonic acid" Spalte 17405, Zusammen- -fassung-Nr. 17 405c**

**PATENT ABSTRACTS OF JAPAN, unexamined applications, Field C, Band 10, Nr. 328, 7.**

**November 1986 THE PATENT OFFICE JAPANESE GOVERNMENT Seite 159 C 383**

(73) Patentinhaber: **LONZA AG
Gampel/Wallis Geschäftsleitung Basel
CH-4002 Basel(CH)**

(72) Erfinder: **Wyss, Heinz, Dr.
Mittelweg 4
Heimberg (Kanton Bern)(CH)**
Erfinder: **Mettler, Hans Peter, Dr.
Kirchweg 16
Brig-Glis (Kanton Wallis)(CH)**
Erfinder: **Previdoli, Felix, Dr.
Rhonesandstrasse 24
Brig (Kanton Wallis)(CH)**

(74) Vertreter: **Weinhold, Peter, Dr. et al
Patentanwälte Dr. V. Schmied-Kowarzik
Dipl.-Ing. G. Dannenberg Dr. P. Weinhold Dr.
D. Gudel Dipl.-Ing. S. Schubert Dr. P. Barz
Siegfriedstrasse 8
W-8000 München 40(DE)**

**Beschreibung**

Die Erfindung betrifft ein Verfahren zur Herstellung von O-substituierten Hydroxylaminen.

Es ist seit langem bekannt, O-substituierte Hydroxylamine aus Hydroxylamin-O-sulfonsäure durch Umsetzung mit einem entsprechenden Alkalialkoholat in Gegenwart eines Alkohols herzustellen (Rendiconti 1965, Vol. 38, S.589 ff, Vol. 39, S.83 ff). Die erreichbaren Ausbeuten nach diesem Verfahren liegen bei maximal 50%.

Von besonderem Nachteil ist ausserdem, dass vielfach nicht umgesetzte, explosive Hydroxylamin-O-sulfonsäure als Alkalisalz im Reaktionsgemisch vorliegt. Die deshalb notwendige Aufarbeitung durch Filtration ist für eine grosstechnische Produktion nicht geeignet. Ausserdem müssten nach diesem Verfahren hergestellte Produkte aufgrund ihrer Unreinheit durch Umkristallisation gereinigt werden.

Aufgabe der vorliegenden Erfindung ist es, diese Nachteile zu vermeiden und ein Verfahren aufzuzeigen, das bessere Ausbeuten liefert und sich zur Umsetzung in die grosstechnische Produktion eignet.

Gelöst werden konnte diese Aufgabe mit einem Verfahren gemäss Patentanspruch 1.

Zur Herstellung von O-substituierten Hydroxylaminen der Formel

$$NH_2 - O - R$$

worin R = $C_1$-$C_6$ Alkyl oder Alkenyl, substituiert oder unsubstituiert, geradkettig oder verzweigt, oder Benzyl, gegebenenfalls substituiert, bedeutet, wird demgemäss Hydroxylamin-O-sulfonsäure mit einem Alkalialkoholat der Formel

$$M - O - R$$

worin M = Natrium oder Kalium bedeutet und R die genannte Bedeutung hat, in Gegenwart eines Alkohols ROH, worin R die genannte Bedeutung hat, und erfindungsgemäss mit einem polaren aprotischen Lösungsmittel als zusätzlichem Lösungsmittel eingesetzt.

Bevorzugte Vertreter von $C_1$-$C_6$-Alkylgruppen sind z.B. die Methyl- oder die Ethylgruppen.

Bevorzugte Vertreter von Alkenylgruppen sind solche mit 1 bis 6 Kohlenstoffatomen, insbesondere die Allyl- oder die Crotylgruppen.

Als Substituenten der Alkyl- oder Alkylengruppen können substituierte Aminogrupen, wie z.B. Dialkylaminogruppen, auftreten. Die Benzylgruppe ist vorzugsweise unsubstituiert. Als mögliche Substituenten können aber Halogenatome wie Chlor und Brom, Nitrogruppen oder Alkylgruppen auftreten.

Die Hydroxylamin-O-sulfonsäure kann auf bekannte Weise aus Hydroxylaminsulfat durch Behandeln mit Oleum zur Verfügung gestellt werden (Inorganic Synthesis V, 122 [1957]).

Als zusätzliche Lösungsmittel sind erfindungsgemäss Tetrahydrofuran, N,N-Dimethylformamid, Acetonitril, Dibutylether, Dimethylacetamid, Dimethylsulfoxid, Sulfolan oder Diethylenglycoldimethylether geeignet. Besonders vorteilhaft wird als zusätzliches Lösungsmittel Acetonitril, Sulfolan oder Dimethylacetamid eingesetzt.

Das Verhältnis zusätzliches Lösungsmittel zu Alkohol liegt insbesondere zwischen 1 : 1 und 10 : 1, vorteilhaft zwischen 3 : 1 und 5 : 1.

Zweckmässig wird so vorgegangen, dass die Hydroxylamin-O-sulfonsäure im zusätzlichen Lösungsmittel vorgelegt wird und darauf das Alkoholat mit dem entsprechenden Alkohol über einen bestimmten Zeitraum zugegeben wird.

Gemäss einer weiteren Variante kann das gelöste Alkalialkoholat gegebenenfalls im zusätzlichem Lösungsmittel vorgelegt und dann die Hydroxylamin-O-sulfonsäure zugegeben werden, und während dieser Zugabe das resultierende O-substituierte Hydroxylamin aus der Reaktion, z.B. durch Destillation entfernt werden.

Für ein Verfahren im industriellen Massstab wird dieser zweiten Variante der Vorzug gegeben.

Es ist ebenfalls möglich, das Alkoholat in situ aus dem entsprechenden Alkalihydroxid MOH und dem Alkohol ROH, worin R und M die genannte Bedeutung haben, zu generieren.

Die Umsetzungstemperatur bewegt sich, abhängig vom Substituenten R, üblicherweise zwischen 0 und 100°C für die bevorzugten Verbindungen mit R = methyl oder Ethyl, vorzugsweise zwischen 15 und 30°C.

Nach einer Reaktionszeit zwischen 2 und 5 Std. kann das Reaktionsprodukt zweckmässig durch Destillation aus dem Reaktionsgemisch abgetrennt werden.

Es ist von Vorteil, das erhaltene O-substituierte Hydroxylamin mit beispielsweise Salzsäure direkt in das entsprechende Hydrochlorid überzuführen und in der Regel als Festprodukt zu isolieren.

Besonders vorteilhaft sind nach dem erfindungsgemässen Verfahren die O-Alkylhydroxylamine, insbesondere das O-Methyl- und O-Ethylhydroxylamin in Ausbeuten bis gegen 80% herstellbar.

Beispiel 1

Herstellung von Methoxyamin-HCl in Acetonitril

Zu 113,09 g (1,0 mol) Hydroxylamin-O-sulfonsäure, suspendiert in 1200 g Acetonitril, tropfte man innert 30 min bei weniger als 10°C eine Lösung von 162,06 g (3,0 mol) Natriummethylat in 378 g Methanol. Die Suspension wurde nun während 3 h bei 20°C gerührt und das entstandene Methoxyamin bei 40°C/200 mbar abdestilliert.
Mit 150 ml HCl konz. stellte man das Destillat auf pH 1, engte ein und trocknete 2 h am Vakuum.
Man erhielt 64,3 g Produkt in einer Ausbeute von 77% (bezogen auf Hydroxylamin-O-sulfonsäure).
Smp.: 145°C

| Elementaranalyse für $CH_5NO.HCl$ | | | |
|---|---|---|---|
| ber. | C 14,4% | H 7,2% | N 16,8% |
| gef. | C 13,8% | H 7,3% | N 17,1% |

Beispiel 2

Herstellung von Methoxyamin-HCl in Dimethylformamid

Zu 113,09 g (1,0 mol) Hydroxylamin-O-sulfonsäure, suspendiert in 1425 g Dimethylformamid, tropfte man innert 30 min eine Lösung von 162,06 g (3,0 mol) Natriummethylat in 378 g Methanol. Diese Suspension wurde nun während 3 h bei 20°C gerührt und das entstandene Methoxyamin bei 40°C/200 mbar abdestilliert.
Mit 150 ml HCl konz. stellte man das Destillat auf pH 1, engte ein und trocknete 2 h am Vakuum.
Man erhielt 36,4 g Produkt in einer Ausbeute von 43% (bezogen auf Hydroxylamin-O-sulfonsäure).

Beispiel 3

Herstellung von Ethoxyamin-HCl

Zu 113,09 g (1,0 mol) Hydroxylamin-O-sulfonsäure, suspendiert in 1582 g Acetonitril, gab man innert 55 min bei weniger als 10°C eine Lösung von 272,2 g (4,0 mol) Natriumethylat in 1008 g Ethanol. Dann rührte man 5 h bei 20°C. Das entstandene Ethoxyamin wurde bei 40°C/200 mbar abdestilliert und mit 150 ml HCl konz. auf pH 1 gestellt.
Nun dampfte man total ein und trocknete 2 h am Vakuum.
Man erhielt 69,6 g Produkt in einer Ausbeute von 71% (bezogen auf Hydroxylamin-O-sulfonsäure). Das Produkt kann zusätzlich durch Umkristallisation aus Ethanol/Acetonitril gereinigt werden.
Smp.: 125-128°C

| Elementaranalyse für $C_2H_7NO.HCl$ | | | |
|---|---|---|---|
| ber. | C 24,6% | H 8,3% | N 14,4% |
| gef. | C 24,3% | H 8,5% | N 14,9% |

Beispiel 4

Herstellung von Isopropoxyamin-HCl

Zu 113,09 g (1,0 mol) Hydroxylamin-O-sulfonsäure, suspendiert in 2500 g Acetonitril, gab man innert 30 min bei weniger als 10°C eine Suspension aus 246,24g (3,0 mol) Natriumisopropylat in 1500 g Isopropanol. Dann rührte man 6 h bei 50-70°C. Das entstandene Isopropoxyamin wurde bei 50°C/200 mbar abdestilliert und mit 66,6 g HCl konz. auf pH 1 gestellt.

Nun dampfte man total ein und trocknete 2 h am Vakuum.

Man erhielt 55,7 g Produkt in einer Ausbeute von 50% (bezogen auf Hydroxylamin-O-sulfonsäure). Das Produkt kann zusätzlich durch Umkristallisation aus Isopropanol/Acetonitril gereinigt werden.

Smp.: 92°C

| Elementaranalyse für $C_3H_9NO.HCl$ | | | |
|---|---|---|---|
| ber. | C 32,3% | H 9,0% | N 12,6% |
| gef. | C 31,9% | H 9,6% | N 13,2% |

Beispiel 5

Herstellung von Crotyloxyamin-HCl

Zu 113,09 g (1,0 mol) Hydroxylamin-O-sulfonsäure, suspendiert in 1582 g Acetonitril, gab man innert 30 min bei weniger als 10°C eine Lösung von 282,27 g (3,0 mol) Natriumcrotylat in 788 g Crotylalkohol. Nun rührte man 3 h bei 30°C. Das entstandene Crotyloxyamin wurde bei 50°C/100 mbar abdestilliert und mit 150 ml HCl konz. auf pH 1 gestellt.

Dann engte man total ein und trocknete 2 h am Vakuum.

Man erhielt 85,5 g Produkt mit einer Ausbeute von 69% (bezogen auf Hydroxylamin-O-sulfonsäure).

Smp.: 178-180°C

| Elementaranalyse für $C_4H_9NO.HCl$ | | | |
|---|---|---|---|
| ber. | C 38,9% | H 8,2% | N 11,3% |
| gef. | C 37,6% | H 8,3% | N 11,6% |

Beispiel 6

Herstellung von Benzyloxyamin-HCl

Zu 113,09 g (1,0 mol) Hydroxylamin-O-sulfonsäure, suspendiert in 500 g Acetonitril, tropfte man innert 1 h bei 20°C eine Lösung von 390,36 g (3,0 mol) Natriumbenzylat in 1960 g Benzylalkohol. Nun rührte man 3 h bei 70°C. Das entstandene Benzyloxyamin wurde bei 50°C/1 mbar abdestilliert und mit 150 ml HCl konz. auf pH 1 gestellt.

Dann engte man total ein und trocknete 2 h am Vakuum.

Man erhielt 69,9 g Produkt in einer Ausbeute von 60% (bezogen auf Hydroxylamin-O-sulfonsäure). Das Produkt kann zusätzlich durch Umkristallisation aus Methanol gereinigt werden.

Smp.: 230-235°C (zers.)

| Elementaranalyse für $C_7H_9NO.HCl$ | | | |
|---|---|---|---|
| ber. | C 52,7% | H 6,3% | N 8,8% |
| gef. | C 52,5% | H 6,4% | N 9,1% |

Beispiel 7

Herstellung von Ethoxyamin-HCL

Eine Lösung von 11,80 g (100 mmol) Hydroxylamin-O-sulfonsäure in 350 ml Ethanol wurde bei 35 bis 45°C und einem Druck von 80 bis 100 mbar innert 2 h zu einer Suspension von 10,20 g (250 mmol) Natriumhydroxid in 20 ml Ethanol und 100 ml Sulfolan gegeben.

Das abdestillierte Gemisch von Ethoxyamin und Ethanol wurde mit 28 g konz. Salzsäure versetzt und eingeengt.
Man erhielt 6,8 g Produkt in einer Ausbeute von 69% (bezogen auf Hydroxylamin-O-sulfonsäure).
Smp.: 125-128°C.

**Patentansprüche**

1.  Verfahren zur Herstellung von O-substituierten Hydroxylaminen der Formel

    $NH_2 - O - R$

    worin R = $C_1$-$C_6$ Alkyl oder Alkenyl, geradkettig oder verzweigt, substituiert oder unsubstituiert, oder Benzyl, gegebenenfalls substituiert, bedeutet, durch Umsetzung von Hydroxylamin-O-sulfonsäure mit einem Alkalialkoholat der Formel

    $M - O - R$

    worin M = Natrium oder Kalium bedeutet und R die genannte Bedeutung hat, in Gegenwart eines Alkohols ROH, worin R die genannte Bedeutung hat, dadurch gekennzeichnet, dass ein polares aprotisches Lösungsmittel als zusätzliches Lösungsmittel eingesetzt wird.

2.  Verfahren nach Patentanspruch 1, dadurch gekennzeichnet, dass sich das Verhältnis von Alkohol zu zusätzlichem Lösungsmittel zwischen 1 : 1 und 1 : 10 bewegt.

3.  Verfahren nach einem oder beiden der Patentansprüche 1 oder 2, dadurch gekennzeichnet, dass als polares aprotisches Lösungsmittel Tetrahydrofuran, N,N-Dimethylformamid, Acetonitril, Dibutylether, Dimethylacetamid, Dimethylsulfoxid, Sulfolan oder Diethylenglycoldimethylether eingesetzt wird.

4.  Verfahren nach einem oder mehreren der Patentansprüche 1 bis 3, dadurch gekennzeichnet, dass eine Reaktionstemperatur zwischen 0 und 100°C eingehalten wird.

5.  Verfahren nach einem oder mehreren der Patentansprüche 1 bis 4, dadurch gekennzeichnet, dass das Alkoholat in situ aus dem Alkalihydroxid MOH und dem Alkohol ROH, worin M und R die genannte Bedeutung haben, erzeugt worden ist.

6.  Verfahren nach einem oder mehreren der Patentansprüche 1 bis 5, dadurch gekennzeichnet, dass das Alkoholat bzw.das entsprechende Alkalihydroxid und der entsprechende Alkohol gegebenenfalls mit dem zusätzlichen Lösungsmittel vorgelegt werden, dann die Hydroxylamin-O-sulfonsäure zugegeben wird und während der Zugabe das resultierende O-substituierte Hydroxylamin aus der Reaktion abgetrennt wird.

7.  Verfahren nach einem oder mehreren der Patentansprüche 1 bis 6, dadurch gekennzeichnet, dass die Hydroxylamin-O-sulfonsäure im zusätzlichen Lösungsmittel vorgelegt wird und erst darauf das entsprechende Alkoholat im Alkohol, gegebenenfalls mit zusätzlichem Lösungsmittel, zugegeben und zur Reaktion gebracht wird.

8.  Verfahren nach einem oder mehreren der Patentansprüche 1 bis 7, dadurch gekennzeichnet, dass die O-substituierten Hydroxylamine durch Zugabe von Salzsäure als Hydrochloride isoliert werden.

**Claims**

1.  Process for preparing O-substituted hydroxyl amines of the formula

    $NH_2 - O - R$

    wherein R is $C_1$-$C_6$-alkyl or alkenyl, straight or branched, substituted or unsubstituted, or benzyl, if desired substituted, by reacting hydroxyl amine-O-sulfonic acid with an alkali alcoholate of the formula

    $M - O - R$

wherein M is sodium or potassium and R has the mentioned meaning, in the presence of an alcohol ROH, wherein R has the mentioned meaning, characterized in that a polar aprotic solvent is used as an additional solvent.

2. Process according to patent claim 1, characterized in that the ratio of the alcohol to the additional solvent is between 1:1 and 1:10.

3. Process according to one or both of patent claims 1 or 2, characterized in that as polar aprotic solvent tetrahydrofurane, N,N-dimethyl formamide, acetonitrile, dibutyl ether, dimethyl acetamide, dimethyl sulfoxide, sulfolane or diethylene glycol dimethyl ether is used.

4. Process according to one or more of patent claims 1 to 3, characterized in that a reaction temperature between 0 and 100°C is maintained.

5. Process according to one or more of patent claims 1 to 4, characterized in that the alcoholate has been prepared in situ from the alkali hydroxide MOH and the alcohol ROH, wherein M and R have the mentioned meanings.

6. Process according to one or more of patent claims 1 to 5, characterized in that the alcoholate or the corresponding alkali hydroxide resp. and the corresponding alcohol are optionally provided with the additional solvent whereupon the hydroxyl amine-O-sulfonic acid is added and the resulting O-substituted hydroxyl amine is separated from the reaction during the addition.

7. Process according to one or more of patent claims 1 to 6, characterized in that the hydroxyl amine-O-sulfonic acid is provided in the additional solvent and afterwards the corresponding alcoholate in the alcohol is added. if desired with additional solvent, and reacted.

8. Process according to one or more of patent claims 1 to 7, characterized in that the O-substituted hydroxyl amines are isolated as hydrochlorides by the addition of hydrochloric acid.

**Revendications**

1. Procédé de préparation d'hydroxylamines O-substituées de formule

NH$_2$ - O - R

dans laquelle R représente un alkyle ou un alcényle en C$_1$-C$_6$ substitué ou non substitué, linéaire ou ramifié, ou un benzyle éventuellement substitué, par réaction de l'acide hydroxylamine-O-sulfonique avec un alcoolate alcalin de formule

M - O - R

dans laquelle M représente le sodium ou le potassium et R a la signification précitée, en présence d'un alcool ROH, dans lequel R a la signification précitée, caractérisé en ce qu'on introduit un solvant aprotique polaire comme solvant additionnel.

2. Procédé selon la revendication 1, caractérisé en ce que le rapport de l'alcool au solvant additionnel varie entre 1:1 et 1:10.

3. Procédé selon l'une ou l'ensemble des revendications 1 ou 2, caractérisé en ce qu'on utilise comme solvant aprotique polaire du tétrahydrofuranne, du N,N-diméthylformamide, de l'acétonitrile, du dibutyléther, du diméthylacétamide, du diméthylsulfoxyde, du sulfolane ou de l'éther diméthylique du diéthylèneglycol.

4. Procédé selon l'une ou plusieurs des revendications 1 à 3, caractérisé en ce qu'on maintient une température de réaction comprise entre 0 et 100°C.

**5.** Procédé selon l'une ou plusieurs des revendications 1 à 4, caractérisé en ce que l'alcoolate a été obtenu in situ à partir de l'hydroxyde alcalin MON et de l'alcool ROH, dans lesquels M et R ont la signification précitée.

**6.** Procédé selon l'une ou plusieurs des revendications 1 à 5, caractérisé en ce qu'on introduit d'abord l'alcoolate ou l'hydroxyde alcalin correspondant et l'alcool correspondant, éventuellement avec le solvant additionnel, qu'on ajoute ensuite l'acide hydroxylamine-O-sulfonique et qu'on sépare de la réaction, pendant l'addition, l'hydroxylamine O-substituée formée.

**7.** Procédé selon l'une ou plusieurs des revendications 1 à 6, caractérisé en ce qu'on introduit d'abord l'acide hydroxylamine-O-sulfonique dans le solvant additionnel et en ce qu'ensuite seulement on ajoute et on fait réagir l'alcoolate correspondant dans l'alcool, éventuellement avec le solvant additionnel.

**8.** Procédé selon l'une ou plusieurs des revendications 1 à 7, caractérisé en ce qu'on isole les hydroxylamines O-substituées sous forme de chlorhydrates par addition d'acide chlorhydrique.